# EUROPEAN PATENT APPLICATION

(11) **EP 1 938 817 A2**
(43) Date of publication of application: **02.07.2008**
(21) Application number: 08102961.3
(22) Date of filing: 21.08.2006
(51) Int. Cl.: A61K 31/315, A61K 33/00, A61K 33/30, A61P 11/06, A61P 17/04, A61P 17/08, A61P 37/08

(54) **Pharmaceutical compositions comprising metals for modulating epithelial cell-to-cell adhesion**

(30) Priority: 19.08.2005 GB 0517043
(62) Divisional of application: 06779604.5
(71) Applicant: York Pharma PLC, 217 Portobello Sheffield, Yorkshire S1 4DP (GB)
(72) Inventor: The designation of the inventor has not yet been filed
(74) Representative: Finnie, Isobel Lara

(57) **Abstract**

A composition comprising a pharmaceutically acceptable group 2, 4, 12, 13 or 14 metal compound for the treatment of a dermatological condition involving an abnormal decrease in the cell-to-cell adhesion between epithelial cells in the epidermal barrier.

## Description

The present invention relates to the treatment, including prophylaxis, of dermatological conditions, compounds and pharmaceutical compositions for use in such treatments and the use of such compounds and compositions for the manufacture of medicaments for use in such treatments. The invention also relates to cosmetic compositions for application to the skin. In particular, the present invention relates to methods of repairing a disease or environmentally damaged skin or epidermal barrier, protecting the skin or epidermal barrier against damage or degradation by disease or environmental factors, compounds and compositions for use in such methods, and the use of such compounds and compositions for the manufacture of medicaments for use in the practice of such methods.

The present invention is particularly useful in the treatment of dermatological conditions involving an abnormal decrease in the cell-to-cell adhesion between epithelial cells, in particular between corneocytes. Examples of such dermatological conditions include various forms of eczema and dermatitis, such as atopic and non-atopic eczema or dermatitis, seborrhoeic eczema, irritant contact dermatitis, allergic contact dermatitis and other sensitive skin conditions, particularly those that cause or are associated with pruritus.

Such conditions, particularly atopic and non-atopic eczema or dermatitis, irritant contact dermatitis and sensitive skin, all arise partially or completely as a result of a defective skin or epidermal barrier.

Atopic eczema and irritant contact dermatitis are both characterised by a chronic phase when the skin is dry and slightly itchy with a defective epidermal barrier. The defective barrier permits the penetration of environmental triggers such as house dust mite faecal allergens and toxins released by the bacterium, Staphylococcus aureus. (Cork 1997 (3); Cork 1996(2)). These allergens trigger a flare of atopic eczema that occurs because the allergens trigger the production of pro-inflammatory cytokines within the skin (Cork 1996 (2); Cork 1999 (5)).

Atopic eczema is a disease that usually starts before the age of two and causes enormous suffering at a crucial time in a child's development. Eczematous skin is characterised by dryness, erythema (redness), exudation and intense pruritus (itching). The itching associated with eczema leads to the child scratching their skin until it bleeds. The prevalence of atopic eczema has been increasing progressively over the past fifty years and now affects up to 30% of children (Williams 1992 (15), Thestrup-Pedersen 1996 (13), Cork et al., 2002 (6)). This rise has been accompanied by an increasing exposure to environmental agents such as soap, detergents and house dust mite (Cork et al., 2002 (6)), each acting to breakdown the skin barrier, an effect more pronounced in patients with atopic eczema (White et al., 1987 (14), Cork 1997 (3)).

The defective epidermal barrier in atopic eczema results in water loss from the corneocytes. The corneocytes shrink and cracks open between them, permitting the penetration of irritants and/or allergens and triggering the development of eczematous lesions.

Atopic eczema is predominantly a disease of childhood and 70% of children will "grow out" of their eczema by the time they are 16. However, in adult life they have a high probability of developing irritant contact dermatitis of the hands, the commonest occupational disease in Europe, as they remain genetically predisposed to barrier breakdown exacerbated by environmental stimuli (soap, detergent etc). The current management of irritant contact dermatitis of the hands consists of irritant avoidance, a complete emollient therapy regimen (Cork 1998 (4)) and topical steroids to treat flare-ups.

Sensitive skin is a condition that affects about 50% of the population, and manifests as burning, stinging and redness following the application of topical products such as cosmetics. Individuals who have sensitive skin may have had a history of atopic eczema.

Atopic eczema is a disease that has increased in prevalence from 4% of children in the 1940's to 30% of children at present. Atopic eczema is an example of multifactorial disease that arises as a result of the interaction of changes in several genes with multiple environmental factors. The genetic basis of atopic eczema has not changed over the past 60 years, but there have been several changes in our environment, these include increased washing with soap and detergents and increased exposure to house dust mites (Cork et al.: 2003 (7)).

The majority of research into the causes of atopic eczema over the past 50 years has focused on the development of IgE mediated allergic responses. However, the majority of children with atopic eczema are not immunologically atopic (Murphy et al., 1999 (12), Flohr et al., 2004 (9)). We have focused, therefore, on the skin or epidermal barrier as a primary site for the development of atopic eczema.

The barrier to the penetration of irritants and allergens into the skin is located in the stratum corneum. At the same time this barrier prevents the loss of water from the host and thereby maintains the internal homeostasis (Cork 1997 (3)). The stratum corneum can be visualised as being rather like a brick wall with the corneocytes forming the bricks and the lamellar lipids the mortar (Elias 1983 (8)).

The corneodesmosomes lock the corneocytes together and prevent shearing forces dislodging the corneocytes (see Figure 1). The corneodesmosomes can be visualised as analogous to the iron rods, which are passed down through holes in bricks to lock them together and add tensile strength to a brick wall.

Corneocytes are shed from the surface of the skin by a process of proteolysis, which is mediated by skin specific proteases, such as the stratum corneum chymotryptic enzyme (SCCE). These proteases are inhibited by skin specific protease inhibitors, such as the secretory leucocyte protease inhibitor (SLPI). It is essential for the process of desquamation to be tightly regulated in order to prevent premature desquamation and a breakdown of the skin barrier. A breakdown/thinning of the stratum corneum will permit the penetration of irritants and allergens, which in turn can lead to the development of flares of atopic eczema.

The integrity of the stratum corneum epidermal barrier is maintained by a balance between the levels of skin proteases, such as SCCE, the protease inhibitors, such as SLPI, and the vulnerability of the adhesion proteins, such as corneodesmosin, to the action of the proteases. The situation is complex as there are more than 10 adhesion proteins, 8 proteases and 10 protease inhibitors.

In normal skin, the breakdown of cellular adhesion proteins (e.g. corneodesmosin) during desquamation is regulated by a balanced expression of proteases (e.g. SCCE) and protease inhibitors (e.g. SLPI). In individuals who are genetically predisposed to atopic dermatitis, there is an increased expression of proteases, such as SCCE, and/or a decreased expression of protease inhibitors, such as SLPI, which leads to a premature breakdown and thinning of the epidermal barrier, allowing the penetration of irritants and allergens.

Atopic eczema, therefore, is a classic example of a gene-environment interaction disease. Multiple environmental factors interact with changes in many genes to produce the disease phenotype. Two of the environmental agents associated with atopic eczema, house dust mites and staphylococcus aureus, produce proteases, which can break the skin barrier down from the outside (because it is a potent immunostimulant, staphylococcus aureus can also induce the production of proteases in the skin). It therefore appears that the skin barrier is being broken down by both endogenous and exogenous proteases in atopic eczema.

The processes involved in irritant contact dermatitis can be essentially the same as those involved in atopic eczema. However, in some cases of irritant contact dermatitis, the abnormal decrease in the cell-to-cell adhesion between the epithelial cells in the epidermal barrier, which underlies the condition, can be caused entirely by an environmental insult, such as exposure to an environmental irritant. In such cases, sufferers need not have had a genetic predisposition towards developing the condition. The processes involved in all of the conditions to which the present invention relates all involve breakdown of the skin or epidermal barrier through similar mechanisms to those involved in both atopic eczema and irritant contact dermatitis.

### Existing treatments for atopic dermatitis or eczema, irritant contact dermatitis and like skin conditions

### (i) Complete Emollient Therapy (best practice)

A regimen consisting of emollient cream/ointments, emollient soap substitutes and bath and shower emollients. These products replace all soap and detergents and as a result produce a reduction of environmental damage to the skin barrier (Cork 1997 (3), Cork 1998 (4), Cork 1999 (5)). Examples of leading emollient products in Europe include: Cream E45, Diprobase®, Hydromol®, Lipobase® and Oilatum®.

Emollients produce a partial repair of the skin barrier but because of the defective corneodesmosomes, associated with these diseases, irritants and allergens can still penetrate through the skin and trigger a flare of the eczema. Moreover, because it involves frequent applications, patients find emollient therapy highly inconvenient and compliance with treatment regimens, therefore, can be poor.

### (ii) Topical corticosteroids

These drugs are used to treat a "flare-up" of atopic eczema. The principle adverse effects of topical steroids are cutaneous atrophy and adrenal axis suppression. These adverse effects are predominantly associated with potent and very potent topical corticosteroids. Steroid paranoia, or steroidphobia, is a significant cause of poor or non-compliance with treatment regimens and is a major problem with all corticosteroids (Charman et al., 2000 (1)).

### (iii) Calcineurin inhibitors, tacrolimus (Protopic®: Fujisawa) and pimecrolimus (Elidel®: Novartis)

These drugs represent a new class of treatment for flare-ups of atopic eczema. They produce a selective inhibition of T cells via the calcineurin pathway. The calcineurin inhibitors do not cause cutaneous atrophy, nor do they have any effect on the adrenal axis. However, whilst treatment with calcineurin inhibitors can control flares of atopic eczema, they do not correct the underlying defects in the epidermal barrier, which allow it to be penetrated by the irritants and allergens that cause such flares.

There is a need, therefore, for new topical products that can repair or prevent damage to the epidermal barrier, for their use would reduce the degree to which it can be penetrated by irritants and allergens, and the frequency or severity of any consequential flares of atopic eczema that require drug treatment.

An object of the present invention is to provide a treatment for dermatological conditions involving an abnormal decrease in the cell-to-cell adhesion between epithelial cells (in particular between corneocytes), especially for eczema, dermatitis and like conditions, such as atopic and non-atopic eczema or dermatitis, seborrhoeic eczema, irritant contact dermatitis, allergic contact dermatitis and other sensitive skin conditions, particularly those that cause or are associated with pruritus, that does not suffer from the drawbacks of the aforementioned known treatments. In particular, it is an object of the invention to provide a treatment that is convenient, does not involve a complex treatment regimen, or the use of drugs with the potential to cause serious side effects of the nature associated with the use of topical corticosteroids.

In accordance with a first aspect of the invention, there is provided a composition comprising a pharmaceutically acceptable group 2, 4, 12, 13 or 14 metal compound for the treatment of a dermatological condition involving an abnormal decrease in the cell-to-cell adhesion between epithelial cells in the epidermal (or skin) barrier, preferably in the stratum corneum.

In a second aspect, the present invention provides a composition comprising a fatty acid, or fatty acid salt or derivative, for the treatment of a dermatological condition involving an abnormal decrease in the cell-to-cell adhesion between epithelial cells in the epidermal (or skin) barrier, preferably in the stratum corneum.

In a third aspect, the present invention provides a composition comprising a pharmaceutically acceptable group 2, 4, 12, 13 or 14 metal compound and a fatty acid, or fatty acid salt or derivative, for the treatment of a dermatological condition involving damage to or degradation of the epidermal (or skin) barrier, preferably in the stratum corneum.

The metal compound is preferably in the form of a particulate solid and it is advantageous for at least about 60, 70, 80 or 90% the particles of the compound to be less than about 5, 2, 1, 0.5 or preferably 0.2µm in size, or for the average (e.g. the mean or median) size of the particles to be less than about 5, 2, 1, 0.5 or preferably 0.2µm. In embodiments, the metal compound can be micronized, or be microfine and the particles of the compound can measure up to about 0.2µm in size. The particle sizes quoted in this specification are in terms of their effective diameters, when measured by techniques conventional in the art.

The metal is preferably zinc and the metal compound is preferably an inorganic salt or an oxide. The most preferred metal compound is zinc oxide. In preferred embodiments, the zinc oxide is micronized or microfine zinc oxide and can consist of particles that measure up to about 0.2µm in size. The zinc oxide can be in the form of the micronized zinc oxide described in US patent no. 5,587,148 (the entire contents of which are incorporated herein by reference) and can be prepared by one of the methods described in this patent. Micornized or microfine zinc oxide with these preferred properties can be used in any aspect of the present invention where the use of a zinc or metal compound is called for.

The fatty acid or fatty acid residue in the fatty acid salt or derivative can contain 4 to 24, preferably 10 to 20, more preferably 14 to 20 and most preferably 16 to 18 carbon atoms. In embodiments, the fatty acid or fatty acid residue is saturated or unsaturated, preferably saturated. In further embodiments, the fatty acid or fatty acid residue is stearic acid or a stearic acid residue, or palmitic acid or a palmitic acid residue. The preferred fatty acid salts are metal stearates and palmitates; the most preferred being zinc stearate and palmitate. The following fatty acids and their residues can be used in the practice of the present invention (in any of its aspects where the use of a fatty acid, salt or derivative is called for): -

| **Common Name** | **Carbon Atoms** | **Chemical Name** | **Type** | **Sources** |
|---|---|---|---|---|
| Palmitic acid | 16 | Hexadecanoic acid | Saturated | Palm oil |
| Palmitoleic acid | 16 | 9-hexadecenoic acid | Unsaturated | Animal fats |
| Stearic acid | 18 | Octadecanoic acid | Saturated | Animal fats |
| Oleic acid | 18 | 9-octadecenoic acid | Unsaturated | Olive oil |
| Linoleic acid | 18 | 9,12-octadecadienoic acid | Unsaturated | Safflower oil |
| Alpha-Linolenic acid | 18 | 9,12,15-octadecatrienoic acid | Unsaturated | Flaxssed (linseed) oil |
| Gamma-Linolenic acid | 18 | 6,9,12-octadecatrienoic acid | Unsaturated | Borage oil |

In accordance with a fourth aspect of the invention, there is provided a composition comprising zinc and a fatty acid residue for use in therapy and preferably for treating a dermatological condition involving damage to or degradation of the epidermal (or skin) barrier, preferably the stratum corneum. The zinc can be in the form of a salt or oxide, and the fatty acid residue can be present as part of a salt or derivative, or the parent fatty acid. The zinc salt or oxide is preferably in the form of a particulate solid and it is advantageous for at least about 60, 70, 80 or 90% the particles of the compound to be less than about 5, 2, 1, 0.5 or preferably 0.2µm in size, or for the average (e.g. the mean or median) size of the particles to be less than about 5, 2, 1, 0.5 or preferably 0.2µm. In embodiments, the zinc salt or oxide is micronized, or microfine and the particles of the compound can measure up to about 0.2µm in size. The fatty acid, or fatty acid residue in the fatty acid salt or derivative, can contain 4 to 24, preferably 10 to 20, more preferably 14 to 20 and most preferably 16 to 18 carbon atoms. In embodiments, the fatty acid or fatty acid residue is saturated or unsaturated, preferably saturated. In further embodiments, the fatty acid or fatty acid residue is stearic acid or a stearic acid residue, or palmitic acid or a palmitic acid residue. The preferred fatty acid salts are metal stearates and palmitates. In an embodiment, the zinc and fatty acid residue form a zinc fatty acid salt, which is preferably zinc stearate or palmitate.

In accordance with a fifth aspect of the invention, there is provided a composition comprising a zinc compound as a microfine particulate solid for use in therapy. The composition is preferably for use in treating a dermatological condition involving damage to or degradation of the epidermal (or skin) barrier, preferably the stratum corneum. At least about 60, 70, 80 or 90% the particles of the zinc compound can be less than about 5, 2, 1, 0.5 or, preferably, 0.2µm in size, or the average (e.g. the mean or median) size of the particles can be less than about 5, 2, 1, 0.5 or, preferably, 0.2µm. In embodiments, the zinc compound is micronized, or microfine and the particles of the compound can measure up to about 0.2µm in size. In embodiments, the zinc compound is a salt or zinc oxide and the composition can further comprise a fatty acid residue. The fatty acid residue can be part of a fatty acid salt or derivative, or can be the parent fatty acid; it preferably contains 4 to 24, 10 to 20, 14 to 20, or 16 to 18 carbon atoms. In embodiments, the fatty acid residue is saturated. Preferably, the fatty acid residue is a stearic acid residue, or a palmitic acid residue. The composition preferably also includes a pharmaceutically acceptable carrier or vehicle and is for topical application to the skin.

In preferred embodiments, compositions in accordance with the invention are formulated for topical application to the skin. In this regard, the inventive compositions can include one or more pharmaceutically acceptable excipient, carrier, diluent or vehicle and can be in the form of a soak, ointment, cream, lotion, paste, gel, stick, spray, aerosol, bath oil, shampoo, soap, foam, spray or solution. Preferred excipients, diluents or vehicles to be included in compositions in accordance with the invention include polyethylene glycol (PEG), including PEG 100, PEG 200, PEG 300 and PEG 400, lower alkyl alcohols, preferably ethanol, light liquid paraffin, Miglyol, isopropyl palmitate, isopropyl myrisitate, jojoba oil, glyceryl stearate, sodium citrate, polysorbate, cetereth 12, phenoxyethanol, methylparaben, propylparaben, disodium cocoamphodiacetate, dehydroacetic acid, silicone fluid, aloe vera oil extract, GPEG-20 stearate, allantoin, carbomer, sodium dehydroacetate, Medilan (hypoallergenic lanolin), shea butter, white soft paraffin, sodium pyrolidone carboxylic acid, yellow soft paraffin, cetomacrogol, isopropyl myristate, water and the like.

Further ingredients that can be included in topically applicable compositions in accordance with the invention include those that assist in the repair of the lipid lamellae, such as ceramides, and compounds found in natural moisturising factor, including sodium pyrolidone carboxylic acid, urea, urocoic acid and lactic acid.

The zinc or other pharmaceutically acceptable group 2, 4, 12, 13 or 14 metal compound is preferably present in compositions in accordance with the invention at a concentration in the range of 0.5 to 75%, 1 to 50%, 4 to 30%, and, more preferably, within the range of 5 to 25% by weight of the composition. The fatty acid, fatty acid salt or derivative can be present in a concentration of 1 to 50, 2 to 30, 5 to 20 and, preferably, 8 to 16 mg/ml of the composition.

In a further aspect, the present invention provides a method for treating a dermatological condition involving damage to or degradation of the epidermal (or skin) barrier, preferably the stratum corneum, comprising administering a composition in accordance with any of the previously described aspects of the invention to a patient suffering from such a dermatological condition.

In a yet further aspect of the invention, there is provided the use of a composition in accordance with any of the first five aspects of the invention in the manufacture of a medicament for the treatment of a dermatological condition involving damage to or degradation of the epidermal (or skin) barrier, preferably the stratum corneum.

The dermatological conditions to be treated in accordance with the various aspects of the invention are preferably those involving an abnormal decrease in the cell-to-cell adhesion between epithelial cells, in particular between corneocytes, in the epidermal (or skin) barrier, particularly in the stratum corneum. The abnormal decrease in the cell-to-cell adhesion between the epithelial cells in the epidermal barrier, which underlies these conditions, can be caused by one or more of many factors. These include genetically determined factors, such as those described above with reference to atopic eczema, and environmental insults, such as exposure to an environmental irritant. Examples of dermatological conditions that can be treated in accordance with the present invention include eczema, dermatitis and like conditions, such as atopic and non-atopic eczema or dermatitis, seborrhoeic eczema, irritant contact dermatitis, allergic contact dermatitis and other sensitive skin conditions, particularly those that cause or are associated with pruritus. The prevention or prophylaxis of dermatological conditions resulting from exposure to an excess of ultraviolet light, including sunburn, are excluded from the ambit of certain preferred embodiments of the present invention, as is the use of any composition in accordance with the invention as a sun screen or for the treatment of diaper rash.

It has recently been suggested that the cutaneous manifestations of atopy, particularly atopic eczema or dermatitis (in the literature, the terms atopic dermatitis and atopic eczema are used interchangeably) often represent the beginning of the atopic march (Spergel and Paller 2003 (16)). It has been shown in several longitudinal studies that approximately half of the infants who suffer from atopic eczema or dermatitis will develop asthma, particularly those with severe atopic eczema, and two-thirds will go on to develop allergic rhinitis (hay fever). Epicutaneous sensitisation is thought to be responsible, with subsequent migration of sensitised T cells into the nose and airways, causing these upper and lower airway diseases. Animal models and human observations concur with this theory and preliminary prevention studies with oral antihistamines have provided evidence that early intervention might slow the atopic march (Spergel and Paller 2003 (16)).

During the first six months of a baby's life, the immune system is 'malleable' and can be more easily switched from TH1 to TH2. It is therefore possible that the defective skin barrier in a baby with atopic eczema or dermatitis is allowing the penetration of allergens at a crucial time, causing TH1 to TH2 switching, and that this immunological change results in a predisposition to more severe allergic eczema, asthma and hay fever. If the skin barrier were to be restored during the first six months of life, therefore, this TH1 to TH2 switching could be prevented or reduced and with it the atopic march and the development of atopic eczema, asthma and hay fever.

Accordingly, in a further aspect of the present invention, compositions in accordance with the invention can be employed in methods of slowing or preventing the atopic march, reducing TH1 to TH2 switching and/or controlling the development, preventing, reducing the risk of development, and/or prophylaxis of upper and lower airways diseases, particularly asthma and allergic rhinitis. In the practice of this method, compositions in accordance with the invention are used to treat atopic eczema or dermatitis, particularly in young children and infants under the age of about 5, 3, 2, 1 or, preferably, six months. Methods of treating airway diseases in this manner also fall within the ambit of the present invention.

In a yet further aspect of the invention, compositions in accordance with the invention can be used as protease antagonists and to thereby prevent or control pruritus. Methods of controlling pruritus in this manner are also within the ambit of the present invention.

In embodiments of the invention, the treatments involve administering a composition in accordance with the invention topically to the skin. The compositions are preferably administered once or twice a day and in an amount sufficient to provide a dose of metal compound or zinc of between 0.01 and 500mg, 0.1 and 100mg, 0.2 and 25mg, 0.3 and 10, and preferably 0.5 and 5mg per day, and/or a dose of fatty acid, fatty acid salt or derivative of between 10 and 1000mg, 25 and 750mg, 50 and 500mg, 75 and 250, and preferably 100 and 150mg per day to each treated area of skin.

In further embodiments, compositions in accordance with the present invention do not include any native or recombinant stratum corneum chymotryptic enzyme (SCCE), or a silicone compound or fluid, particularly in a pharmaceutically effective amount. In other preferred embodiments, the metal compound is not zinc sulphate and the zinc is not present in the form of zinc sulphate.

In embodiments, it can be advantageous for at least about 60, 70, 80 or 90% the particles of the particulate solid, or zinc salt, compound or oxide in a composition to be greater than about 0.02, 0.05 or 0.1µm in size, or for the average (e.g. the mean or median) size of the particles to be greater than about 0.02, 0.05 or 0.1 µm in size.

In this specification, the groups in the periodic table are identified using the current (new) IUPAC notation, wherein groups 2, 4, 12, 13 and 14 correspond to groups IIa, IVb, IIb, IIIa and IVa in the CAS system.

When used in this specification to qualify the nature of a compound or composition, the term "pharmaceutically acceptable" means that the compound, or composition, in question does not cause significant side effects in a significant number of patients, when it is used in a quantity, or concentration at which it has the promised therapeutic effect. All references in this specification to the treatment of diseases, conditions and the like encompass prophylaxis or preventative treatments, as well as therapeutic or curative use.

The following examples are provided by way of illustration only and are not intended to in any way limit the scope of the present invention.

### Example 1

### The topical corticosteroid induced protease skin barrier damage model

Topical glucocorticoids (corticosteroids), which are used in the treatment of atopic dermatitis, damage the skin barrier and cause changes to the lipids within the epidermis, and a reduction in the number corneodesmosomes holding the corneocytes together. Corticosteroids have also been shown to up-regulate the expression of KLK7, a gene that encodes the stratum corneum chymotryptic enzyme (SCCE), which is a major protease involved in desquamation. Therefore, skin that has been pretreated with gluticocorticoids can provide a good model in which to test the efficacy of compounds for their capacity to restore the skin or epidermal barrier and to be useful in the treatment of dermatological conditions that compromise the skin or epidermal barrier, particularly those involving an abnormal decrease in the cell-to-cell adhesion between epithelial cells (in particular between corneocytes) such as atopic eczema or dermatitis, sebarrhoeic eczema, irritant contact dermatitis, allergic contact dermatitis and other sensitive skin conditions.

Kao et al (2003) (reference (11)) showed that the capacity of gluticocorticoids to compromise the permeability and integrity of the skin barrier could be demonstrated by using tape stripping followed by measurement of transepidermal water loss (TEWL), and the amount of protein removed per stripping. The techniques disclosed by Kao et al. provide a measure of barrier integrity and corneocyte cohesion (any increases in TEWL are indicative of a loss in barrier function and increases in total protein removed by the skin strips are the result of a loss of intercellular cohesion, and *vice versa).* The methods employed by Kao were therefore used in the following examples. The TEWL measurements were taken using an "Aquaflux AF 102" electrolytic water analyser (Available form Biox Systems Ltd, Southwark Campus, 103 Borough Road, London SE1 0AA, UK) in the manner described in Berg et al. (Berg, E. P. Pascut, F. C., Ciortea, L. I., O'Driscoll, D., Xiao, P. and Imhof, R. E. (2002), Aquaflux - A new instrument for water vapour flux density measurement Proceedings of the 4th International Symposium on Humidity and Moisture, Centre for Measurement Standards, ITRI, RoC, ISBN 957-774-423-0, pp288-95) and Ciortea et al. (Ciortea, L. I. O'Driscoll, D., Berg, E. P., Xiao, P., Pascut, F. C. and Imhof, R. E. (2002), New methods for measuring water desorption and vapour permeation rates in membranes Proceedings of the 4th International Symposium on Humidity and Moisture, Centre for Measurement Standards, ITRI, RoC, ISBN 957-774-423-0, pp288-95).

### Example 1a

Microfine zinc oxide (available from BASF Aktiengesellschaft, 67056 Ludwigshafen, Germany, under the trade mark Z-Cote®, item no. 55082355, or from Univar, Basildon, Essex, UK, as item no. 76011010; see also: Z-Cote®, BASF; Mitchnick, M. A., Fairhurst, D. and Pinnell, S. R. (1999) J Am Acad Dermatol 40: 85-90) (25%) in light liquid paraffin was applied twice a day to a volunteer's left forearm for 4 days; his right forearm received light liquid paraffin only. One fingertip unit of clobetasol propionate (a potent corticosteroid) was then applied twice a day to the study areas for 3 days. TEWL was then measured at base line and following the removal of 4, 8, 12 and 16 tape strips. The results of this experiment, which are set out in **Figure 2** (Z denotes the zinc oxide formulation in this figure), showed a 37.5% reduction in TEWL following the removal of 16 tape strips, in skin that had been pretreated with the microfine zinc oxide preparation.

### Example 1b

Microfine zinc oxide, 5 and 15%, in light liquid paraffin was applied twice a day to a volunteer's the left bicep and left forearm for 4 days, respectively; the right bicep received light liquid paraffin only. One fingertip unit of clobetasol propionate was then applied twice a day to the study areas for 3 days (clobetasol was applied in combination with the zinc oxide preparation and light liquid paraffin for the last day of treatment with these agents). TEWL was then measured at base line and following the removal of 4, 8, 12 and 16 tape strips. The results of this study are set out in **Figure 3** and show that the areas treated with 5 and 15% zinc oxide preparations exhibited a dose-dependant response with respect to the protective effect afforded by the zinc oxide. In **Figure 3**, RFA stands for right forearm, LB stands for left bicep, RB stands for right bicep, Z stands for the zinc oxide preparation and LLP stands for light liquid paraffin (used alone).

### Example 1c

Four finger dips of 12mg/ml zinc stearate in light liquid paraffin were applied to the right forearm for 9 days, twice daily. The left forearm received light liquid paraffin only. On day 6 through 9, one fingertip unit of clobetasol propionate was applied to the left and right forearms and the right bicep, twice a day. On the days where both the test preparations and the clobetasol were applied, the test formulations were applied at least one hour before the steroid. The preparations were allowed to soak into the skin for one hour. Any residue left after this period was blotted off using a KIM-wipe. On day 10, TEWL was measured at base line following the removal of 4, 8, 12 and 16 tape strips. The results of this study are set out in **Figure 4** and they demonstrate that zinc stearate can also protect against the effects of clobetasol. In Figure 4, LFA stands for left forearm, RFA for right forearm, RB for right bicep, ZS stands for zinc stearate in light liquid paraffin, and LLP stands for light liquid paraffin (used alone).

### Example 1d

For a period of 4 days two finger dip units of PEG400 in ethanol (7 parts PEG and 3 parts ethanol) were applied 6 times a day for 4 days to the left forearm, two finger dip units of 12mg/ml palmitic acid in PEG400 and ethanol (7 parts PEG and 3 parts ethanol) were applied 6 times a day for 4 days to the right forearm. Thereafter, the right and left forearms and the left bicep were treated with one finger unit of clobetasol propionate applied 3 times over the course of 24 hours. TEWL was then performed at base line and after 4, 8, 12, 16 and 20 tape strips. The results of this study are shown in **Figure 5**, where it can be seen that the rate of TEWL increase during tape stripping was significantly reduced in skin treated with palmitic acid when compared to control and vehicle alone. In **Figure 5**, LFA stands for left forearm, RFA for right forearm, LB for left bicep, and P stands for the palmitic acid preparation.

### Example 1e

For a period of 4 days two finger dip units of PEG400 in ethanol (7 parts PEG and 3 parts ethanol) were applied 6 times a day to the right forearm, two finger dip units of microfine zinc oxide (25%) in PEG400 and ethanol (7 parts PEG and 3 parts ethanol) were applied 6 times a day to the left forearm, and two finger dip units of 12mg/ml palmitic acid and microfine zinc oxide (25%) in PEG400 and ethanol (7 parts PEG and 3 parts ethanol) were applied 6 times a day to the right bicep. Thereafter, both forearms and biceps were treated with one finger unit of clobetasol propionate applied 3 times over the course of 48 hours. During the two days when the clobetasol was applied, the microfine zinc oxide alone or microfine zinc oxide combined with palmitic acid were applied only three times per day, and at different times to the clobetasol, in order to reduce the chance of an interaction. TEWL was then performed at base line and after 4, 8, 12, 16 and 20 tape strips. The results of this study are shown in **Figure 6**, where it can be seen that the rate of TEWL increase during tape stripping was significantly reduced in skin treated with zinc oxide, and zinc oxide and palmitic acid, when compared to control and vehicle alone. In **Figure 6**, LFA stands for left forearm, RFA for right forearm, LB for left bicep, RB for right bicep, Z stands for zinc oxide and P stands for palmitic acid.

### Example 2

### Skin stripping protease induced skin barrier damage model

It has recently been demonstrated that tape stripping alone induces the production of proteases in the skin and that if the skin is treated with buffers, which alter the skin pH towards acid (pH4.5), the rate of barrier recovery over the next 72 hours is increased (See Hachem et al., 2005). Tape stripping alone, therefore, represents a good model in which to test the efficacy of compounds for their capacity to restore the skin or epidermal barrier and to be useful in the treatment of dermatological conditions that compromise the skin or epidermal barrier, particularly those involving an abnormal decrease in the cell-to-cell adhesion between epithelial cells (in particular between corneocytes) such as atopic eczema or dermatitis, seborrhoeic eczema, irritant contact dermatitis, allergic contact dermatitis and other sensitive skin conditions. This model of protease induced skin barrier damage is also much simpler than the topical corticosteroid model and was used in this example.

Prior to treatment, the test skin areas underwent tape stripping sufficiently to provide a TEWL of greater than 50g/m²hr. Two finger units of test formulation were then applied to the tape stripped skin. TEWL was measured 3 hours after tape stripping was completed, in order to provide a base line reading, and then 24 hours post tape stripping. **Figure 7** shows the percentage recovery, in terms of TEWL reduction, over the 21 hour period between the two TEWL measurements resulting from a formulation consisting of 25% zinc oxide suspended in a 70% PEG400/30% ethanol mixture (Z in **Figure 7**)**,** 12mg/ml of palmitic acid in the same vehicle (P in **Figure 7**) and a composition consisting of 25% zinc oxide and 12mg/ml palmitic acid in the same vehicle (25% Z 12mg/ml P in **Figure 7**)**.** The data shown in **Figure 7** has been corrected to account for the positive effect of the vehicle (PEG400) and demonstrates that, when used together, palmitic acid and zinc oxide have a more than additive, that is to say a synergistic, effect on inducing skin barrier recovery. The zinc oxide employed in this study was micro-fine.

### Example 3

Topically applicable formulations in accordance with the invention can be prepared by mixing the following ingredients using conventional techniques and apparatus of a nature that would be well known to those skilled in the art. The microfine zinc oxide used in these formulations is Z-Cote®, Z-Cote® HP1, or Z-Cote® MAX™, all of which are available from BASF Aktiengesellschaft, 67056 Ludwigshafen, Germany. Z-Cote® HP1, or Z-Cote® MAX™ consist of microfine zinc oxide coated with 2% of a hydrophobic silicone material (Z-Cote® HP1) or between 1 and 4% of a polar/hydrophobic silicone material (Z-Cote® MAX™). Z-Cote® is uncoated microfine zinc oxide. All grades of Z-Cote® can be used in the practice of the present invention.

### Formulation 1

Microfine zinc oxide 5%
Palmitic acid 2%
Light liquid paraffin 10%
Polyethylene glycol 20%
Water 63%

### Formulation 2

Microfine zinc oxide 10%
Palmitic acid 2%
Light liquid paraffin 10%
Polyethylene glycol 20%
Water 58%

### References:

(1) Charman CR, Morris AD, Williams HC. Topical corticosteroid phobia in patients with atopic eczema. Br J Dermatol 2000; 142: 931-6
(2) Cork M.J. (1996) The role of staphylococcus aureus in atopic eczema: treatment strategies. JEADV 7(7): S31-S37.
(3) Cork MJ. The importance of skin barrier function. J Dermatol Treat 1997; 8: S7-S13
(4) Cork MJ. Comkplete Emollient Therapy. The National Association of Fundholding Practices Year book 1998, pp 159-68.
(5) Cork MJ, Butler L, Young S et al. An audit of the effect of explanation and demonstration of topical therapy for atopic eczema by specialist nurses. Brit J Dermatol 1999; 141 (Suppl 55): 102-7.
(6) Cork MJC, Murphy R, Carr J, Buttle D, Ward S, Båvik C and Tazi-Ahnini R. The rising prevalence of atopic eczema and environmental trauma to the skin. Dermatology in Practice 2002; 10 (3): 22-26
(7) Cork M.J., Timmins J., Holden C., Carr J. Berry V., Ward S.J., Tazi-Ahnini R. (2003) An audit of adverse drug reactions to aqueous cream in children with atopic eczema. Pharmaceutical Journal 271 : 746-747
(8) Elias PM. Epidermal lipids, barrier function and desquamation. J. Invest. Dermatol. 1983; 80: (6) 44-49.
(9) Flohr C, Johansson SG, Wahlgren CF, Williams H. How atopic is atopic dermatitis? J Allergy Clin Immunol. 2004 Jul; 114(1): 150-8.
(10) Jean-Pierre Hachem*, Mao-Quiang Man*, Debra Crumrine*, Yoshikazu Uchida*, Barbara E. Brown*, Vera Rogiers, Diane Roseeuw, Kenneth R. Feingold and Peter M. Elias*. "Sustained Serine Proteases Activity by Prolonged Increase in pH Leads to Degradation of Lipid Processing Enzymes and Profound Alterations of Barrier Function and Stratum Corneum Integrity" Journal of Investigative Dermatology, June 2005.
(11) Kao JS, Fluhr JW, Man MQ et al. Short-term glucocorticoid treatment compromises both permeability barrier homeostasis and stratum corneum integrity: inhibition of epidermal lipid synthesis accounts for functional abnormalities. J Invest Dermatol 2003; 120: 456-464.
(12) Murphy R, Williams HC, Duff GW, Cork MJ, Total and specific IgE and definitions of atopy. Br. J. Dermatol. 141 (suppl.):25, 1999.
(13) Thestrup-Pedersen K. The incidence and pathophysiology of atopic dermatitis. J Eur Acad Dermatol Venereol 1996; 7 (suppl 1): 53-57.
(14) White MI, McEwan Jenkinson D, Lloyd DH. The effect of washing on the thickness of the stratum corneum in normal and atopic individuals. Br. J. Dermatol. 1987 116: 525-30.
(15) Williams HC. Is the prevalence of atopic dermatitis increasing? Clinical and Experimental Dermatology 1992; 17: 385-391
(16) Spergel JM and Paller AS. Atopic dermatitis and the atopic march. J Allergy Clin. Immunol. Vol 112, No. 6 (2003)

## Claims

1. A composition comprising a pharmaceutically acceptable group 12, 2, 4, 13 or 14 metal compound for the treatment of a dermatological condition involving an abnormal decrease in the cell-to-cell adhesion between epithelial cells in the epidermal barrier.

2. A composition comprising a fatty acid, or fatty acid salt or derivative, for the treatment of a dermatological condition involving an abnormal decrease in the cell-to-cell adhesion between epithelial cells in the epidermal barrier.

3. A composition comprising a pharmaceutically acceptable group 12, 2, 4, 13 or 14 metal compound and a fatty acid, or fatty acid salt or derivative, for the treatment of a dermatological condition involving damage to or degradation of the skin or epidermal barrier.

4. A composition as claimed in claim 3, wherein the dermatological condition involves an abnormal decrease in the cell-to-cell adhesion between epithelial cells in the epidermal barrier.

5. A composition as claimed in any of claims 1, 3 or 4, wherein the metal compound is a particulate solid, optionally wherein
a) at least about 60, 70, 80 or 90% the particles of the compound are less than about 5, 2, 1, 0.5 or 0.2µm in size, and/or
b) the average size of the particles of the compound is less than about 5, 2, 1, 0.5 or 0.2µm.

6. A composition as claimed in any of claims 1, and 3-5, wherein the metal is zinc.

7. A composition as claimed in any of claims 1, and 3-6, wherein the metal compound is an inorganic salt or an oxide.

8. A composition as claimed in claim 6 or 7, wherein the compound is zinc oxide.

9. A composition as claimed in any of the preceding claims, said composition comprising a fatty acid, or fatty acid salt or derivative, wherein the fatty acid or fatty acid residue in the fatty acid salt or derivative contains 4 to 24, 10 to 20, 14 to 20 or 16 to 18 carbon atoms,
preferably wherein the fatty acid or fatty acid residue is saturated,
more preferably wherein the fatty acid or fatty acid residue is stearic acid or a stearic acid residue, or palmitic acid or a palmitic acid residue,
still more preferably wherein the fatty acid salt is a metal stearate or palmitate and even more preferably wherein the fatty acid salt is zinc stearate or palmitate.

10. A composition comprising zinc and a fatty acid residue for use in therapy.

11. A composition as claimed in claim 10, wherein the composition is for treating a dermatological condition involving damage to or degradation of the epidermal barrier, optionally wherein the dermatological condition involves an abnormal decrease in the cell-to-cell adhesion between epithelial cells in the epidermal barrier.

12. A composition as claimed in any of claims 10-11, wherein the zinc is in the form of a salt or oxide.

13. A composition as claimed in any of claims 10-12, wherein the fatty acid residue is part of a fatty acid salt or derivative, or the parent fatty acid.

14. A composition as claimed in any of claims 10-13, wherein the zinc is in the form of a salt or oxide and is a particulate solid, optionally wherein at least about 60, 70, 80 or 90% the particles of the particulate solid are less than 5, 2, 1, 0.5 or 0.2µm in size, or the average size of the particles of the particulate solid is less than about 5, 2, 1, 0.5 or 0.2µm.

15. A composition as claimed in any of claims 10-14, wherein the fatty acid, or fatty acid residue in the fatty acid salt or derivative, contains 4 to 24, 10 to 20, 14 to 20, or 16 to 18 carbon atoms.

16. A composition as claimed in any of claims 10-15, wherein the fatty acid or fatty acid residue is saturated,
optionally wherein the fatty acid or fatty acid residue is stearic acid or a stearic acid residue, or palmitic acid or a palmitic acid residue and
preferably wherein the fatty acid salt is a metal stearate or palmitate.

17. A composition as claimed in any of claims 10-16, wherein the zinc and fatty acid residue form a zinc fatty acid salt,
optionally wherein the zinc fatty acid salt is zinc stearate or palmitate.

18. A composition comprising a zinc compound as a microfine particulate solid for use in therapy.

19. A composition as claimed in claim 18, for treating a dermatological condition involving damage to or degradation of the epidermal barrier,
optionally wherein the dermatological condition involves an abnormal decrease in the cell-to-cell adhesion between epithelial cells in the epidermal barrier.

20. A composition as claimed in any of claims 18-19, wherein at least about 60, 70, 80 or 90% the particles of the compound are less than about 5, 2, 1, 0.5 or 0.2µm in size, or the average size of the particles is less than about 5, 2, 1, 0.5 or 0.2µm.

21. A composition as claimed in any of claims 18-20, wherein the zinc compound is a salt or zinc oxide.

22. A composition as claimed in any of claims 18-21, further comprising a fatty acid residue,
optionally wherein and the fatty acid residue is part of a fatty acid salt or derivative, or the parent fatty acid.

23. A composition as claimed in claim 21, wherein the fatty acid residue contains 4 to 24, 10 to 20, 14 to 20, or 16 to 18 carbon atoms,
optionally wherein the fatty acid residue is saturated,
preferably wherein the fatty acid residue is a stearic acid residue, or a palmitic acid residue.

24. A composition as claimed in any of the preceding claims further comprising an ingredient that assists in the repair of the lipid lamellae,
optionally further comprising a ceramide, sodium pyrolidone carboxylic acid, urea, urocoic acid or lactic acid.

25. A composition as claimed in any of the preceding claims, for the treatment of a) eczema, dermatitis or a like condition, and/or
b) atopic eczema, non-atopic eczema, seborrhoeic eczema, irritant contact dermatitis, allergic contact dermatitis or pruritus.

26. A composition as claimed in any of the preceding claims, wherein, when present, the zinc, zinc compound or other group 12, 2, 4, 13 or 14 metal compound is in a concentration of 0.5 to 75%, 1 to 50%, 4 to 30%, or 5 to 25% by weight of the composition.

27. A composition as claimed in any of the preceding claims, wherein, when present, the fatty acid residue, fatty acid, fatty acid salt or fatty acid derivative is in a concentration of 1 to 50, 2 to 30, 5 to 20, or 8 to 16 mg/ml of the composition.

28. A composition as claimed in any of the preceding claims formulated for topical application to the skin.

29. A composition as claimed in any of the preceding claims, further comprising one or more pharmaceutically acceptable excipient, carrier, diluent or vehicle.

30. A composition as claimed in any of the preceding claims, for the slowing, prevention or prophylaxis of the atopic march, and/or reducing TH1 to TH2 switching,
optionally for use in controlling the development, preventing, reducing the risk of development, or prophylaxis of asthma and/or allergic rhinitis.

31. A composition as claimed in any of the preceding claims, wherein the treatment involves topical application of the composition to the skin.

32. A composition as claimed in any of the preceding claims, wherein the treatment is prophylactic.

33. A composition as defined in any one of the preceding claims for use in a method of treating a dermatological condition involving damage to or degradation of the epidermal or skin barrier, comprising administering the composition to a patient suffering from such a dermatological condition.

34. A composition as claimed in claim 33, wherein the dermatological condition involves an abnormal decrease in the cell-to-cell adhesion between epithelial cells, preferably wherein the epithelial cells are corneocytes.

35. A composition as defined in any one of claims 1-32 for use in a method of for slowing, preventing or the prophylaxis of the atopic march, and/or reducing TH1 to TH2 switching, comprising administering the composition to a patient suffering from a dermatological condition.

36. A composition as defined in any one of claims 1-32 for use in a method for use in controlling the development, preventing, reducing the risk of development, or prophylaxis of asthma and/or allergic rhinitis, comprising administering the composition to a patient suffering from a dermatological condition.

37. A composition as claimed in any of claims 33-36, wherein the dermatological condition is atopic eczema, non-atopic eczema, seborrhoeic eczema, irritant contact dermatitis, allergic contact dermatitis or pruritus.

38. A composition as claimed in any of claims 33-37, wherein the composition is applied topically to the skin,
preferably wherein the compositions are administered once or twice a day.

39. A composition as claimed in any of claims 33-38, wherein the treatment is prophylactic.
